# EUROPÄISCHE PATENTANMELDUNG

(11) **EP 4 749 271 A1**
(43) Veröffentlichungstag der Anmeldung: **27.05.2026**
(21) Anmeldenummer: 24214801.3
(22) Anmeldetag: 22.11.2024
(51) Int. Cl.: G01N 25/48, A61L 2/20

(54) **WASSERSTOFFPEROXIDSENSOR UND VERFAHREN ZUR BESTIMMUNG EINER KONZENTRATION VON WASSERSTOFFPEROXID IN EINEM MESSGAS**

(71) Anmelder: SKAN AG, 4123 Allschwil (CH)
(72) Erfinder: Lehmann, Frank, 4102 Binningen (CH); Sommerhalder, Matthias, 4112 Bättwil (CH); Kemmerling, Dr. Simon, 4106 Therwil (CH); Krebsbach, Dr. Timo, 4310 Rheinfelden (CH); Eggert, Benjamin, 02763 Zittau (DE); Novák, Martin, 4102 Binningen (CH); Hommes, Dr. Gregor, 47669 Wachtendonk (DE)
(74) Vertreter: Mertzlufft-Paufler, Cornelius

(57) **Zusammenfassung**

Die Erfindung schlägt einen Wasserstoffperoxidsensor (1) vor, welcher einen passiven Messsensor (2) und einen passiven Referenzsensor (3) umfasst. Diese Sensoren sind mit einem Messgas in Kontakt bringbar. Der Messsensor (2) ist, beispielsweise durch eine katalytisch wirksame Schicht (8), zur katalytischen Umsetzung von Wasserstoffperoxid (5) eingerichtet. Dabei ist der Messsensor (2) als ein Temperatursensor (6) ausgebildet. Damit kann der Messsensor (2) die entstehende Wärme bei Zersetzung des Wasserstoffperoxids (5) an dem Messsensor (2) detektieren.

## Beschreibung

Die Erfindung betrifft einen Wasserstoffperoxidsensor und ein Verfahren zur Bestimmung einer Konzentration von Wasserstoffperoxid in einem Messgas. Insbesondere betrifft die Erfindung einen Wasserstoffperoxidsensor und ein Verfahren zur Anwendung im Bereich pharmazeutischer und/oder biotechnologischer Anlagen.

### Hintergrund

In pharmazeutischen Produktionsanlagen ist die Dekontamination von Isolatoren von entscheidender Bedeutung, um die Sterilität und damit die Qualität und Sicherheit der hergestellten Produkte zu gewährleisten. Isolatoren schützen sensible Produktionsbereiche vor Kontaminationen durch die Umgebungsluft und das Personal. Eine effektive Dekontaminationsmethode in diesen Anlagen ist die Verwendung von Wasserstoffperoxid (H₂O₂), das in Form von Dampf eingesetzt wird. H₂O₂ ist ein starkes Oxidationsmittel, das in der Lage ist, eine Vielzahl von Mikroorganismen, einschließlich Bakterien, Viren und Pilzen, effizient abzutöten, indem es ihre Zellstrukturen zerstört.

Die Dekontamination mit H₂O₂ erfolgt in einem kontrollierten Prozess, bei dem der Dampf in den Isolator eingebracht wird, um alle Oberflächen und die Luft gründlich zu desinfizieren. Dieser Prozess erfordert genaue Überwachung und Steuerung, da eine unzureichende H₂O₂-Konzentration zu einer unvollständigen Dekontamination führen kann, während eine zu hohe Konzentration potenziell schädlich für Materialien und die Sicherheit der Mitarbeiter sein kann.

Um die Wirksamkeit und Sicherheit des Dekontaminationsprozesses zu gewährleisten, werden H₂O₂-Sensoren eingesetzt. Diese Sensoren überwachen kontinuierlich die Konzentration des Wasserstoffperoxids im Isolator, um sicherzustellen, dass die festgelegten Dekontaminationsparameter eingehalten werden. Zudem spielen sie eine wichtige Rolle bei der Kontrolle des Abbaus von H₂O₂ nach Abschluss des Prozesses, um sicherzustellen, dass keine gefährlichen Rückstände verbleiben, die den Produktionsprozess oder das Produkt beeinträchtigen könnten. Durch den Einsatz dieser Sensoren wird die Dekontamination präzise, sicher und zuverlässig durchgeführt, was entscheidend für die Einhaltung der hohen Standards in der pharmazeutischen Produktion ist.

Der vorliegenden Erfindung liegt daher die Aufgabe zugrunde, Vorrichtungen und Verfahren zur verbesserten Dekontamination kontrollierter Umgebungen mittels H2O2 bereitzustellen.

### Zusammenfassung der Erfindung

Die Lösung dieser Aufgabe besteht in den Merkmalen von Anspruch 1 und somit bei einem Wasserstoffperoxidsensor der eingangs erwähnten Art insbesondere darin, dass der Wasserstoffperoxidsensor einen passiven Messsensor und einen passiven Referenzsensor umfasst, wobei der Messsensor und der Referenzsensor mit einem Messgas in Kontakt bringbar ist und wobei der Messsensor zu einer katalytischen Umsetzung von Wasserstoffperoxid eingerichtet ist. Dabei ist insbesondere zumindest der Messsensor als ein Temperatursensor ausgebildet.

Ein solcher Wasserstoffsensor erlaubt eine kalorimetrische Messung der Konzentration von Wasserstoffperoxid, insbesondere der Konzentration von H2O2 in einem Messgas, beispielsweise in einer kontrollierten Umgebung. Das Messgas kann dabei die Atmosphäre bzw. Luft innerhalb der kontrollierten Umgebung sein. Die kontrollierte Umgebung kann dabei beispielsweise ein Isolator, ein Reinraum oder eine Containment-Anlage sein. Insbesondere kann die H2O2 Konzentration in einem Bereich zwischen 0 und 1500 ppm präzise bestimmt werden. Der Wasserstoffsensor ist besonders effektiv bei Konzentrationen um 700 ppm. Er eignet sich zudem für Umgebungen bzw. in Messgasen mit Temperaturen um beispielsweise 15-40 °C, da bei diesen Bedingungen die Aufnahmefähigkeit der Luft für Wasserstoffperoxid deutlich geringer ist, was die Messgenauigkeit weiter erhöht.

In einer Ausführungsform ist der Referenzsensor als ein Temperatursensor ausgebildet.

Hierbei kann ein passiver Messsensor und/oder ein passiver Referenzsensor beispielsweise dadurch charakterisierbar sein, dass ein Betrieb bei einer vorliegenden Umgebungstemperatur durchführbar ist. Insbesondere kann somit erreichbar sein, dass der Messsensor und/oder der Referenzsensor im Betrieb nicht oder zumindest nicht nennenswert auf ihre jeweilige Umgebung thermisch einwirken. Eine unerwünschte oder unkontrollierte Aufheizung von Komponenten, beispielsweise anderen Sensoren wie beispielsweise einem Feuchtesensor, derart, dass deren bestimmungsgemäße Funktion beeinträchtig wird, lässt sich so vermeiden. Im Ergebnis ist es somit möglich, eine Anordnung auf sehr kleinem Bauraum zu realisieren.

Der Einsatz eines Temperatursensors als Referenzsensors bietet den Vorteil, externe Einflüsse wie beispielsweise die Feuchtigkeit zu kompensieren. Dadurch können Messabweichungen erkannt und die Genauigkeit der Messungen erhöht werden. Der Referenzsensor hilft somit, zuverlässigere und präzisere Messergebnisse zu gewährleisten.

In einer weiteren Ausführungsform ist vorgesehen, dass der Messsensor und der Referenzsensor baugleiche Sensorelemente aufweisen.

Es ist besonders vorteilhaft, wenn der Referenzsensor baugleiche Elemente wie der Messsensor aufweist, weil dies die Vergleichbarkeit und Konsistenz der Messungen erhöht. Baugleiche Sensoren reagieren identisch auf dieselben Umwelteinflüsse und Betriebsbedingungen. Dies bedeutet, dass eventuelle Abweichungen oder Drifts in der Sensorleistung konsistent zwischen dem Messsensor und dem Referenzsensor sind. Dadurch können präzisere Kalibrierungen und Anpassungen vorgenommen werden, was zu genaueren und verlässlicheren Messresultaten führt.

In einer Ausführungsform ist der Messsensor zusätzlich mit einer katalytisch wirksamen Schicht abgedeckt.

Die Verwendung einer katalytischen Schicht auf dem Messsensor ermöglicht die kalorimetrische Bestimmung der Wasserstoffperoxid-Konzentration. Insbesondere kann der Temperatursensor selbst mit einer katalytischen Schicht beschichtet werden. Diese Beschichtung ermöglicht es dem Sensor, die Konzentration von Wasserstoffperoxid durch die katalytische Reaktion mit dem Wasserstoffperoxid zu messen, was zu präzisen und verlässlichen Ergebnissen führt.

In einer Ausführungsform weist der Messsensor oder der Referenzsensor ein volumenhaltiges Sensorelement auf.

Die Verwendung eines volumenartigen Sensorelements bietet eine robuste Lösung, die insbesondere gegenüber Feuchteinflüssen unempfindlich ist. Im Gegensatz dazu können bei flächenmäßigen Sensoren Feuchtigkeitseinwirkungen zu einem Kurzschluss der Mäander führen, wodurch deren Empfindlichkeit gegenüber Umgebungsfeuchte beeinträchtigt wird. Die robuste volumenhaltige Anordnung gewährleistet somit eine zuverlässige Leistung auch unter feuchten Bedingungen.

In einer Ausführungsform weisen der Messsensor und der Referenzsensor ein volumenhaltiges Sensorelement auf.

Weisen sowohl der Messsensor als auch der Referenzsensor ein volumenhaltiges Sensorelement auf, werden im Vergleich zur Nutzung bei nur einem der Sensoren, hierzu genannte Vorteile weiter verstärkt. Weiterhin bleibt die Vergleichbarkeit der Sensoren gegeben.

In einer weiteren alternativen Ausführungsform weist der Messsensor oder der Referenzsensor ein flächiges Sensorelement auf.

Die Verwendung eines flächigen Sensorelements bietet den Vorteil, dass ihre größere Oberfläche die Empfindlichkeit und Signalqualität erhöht, da mehr Daten erfasst und verarbeitet werden können. Flächige Sensorelemente ermöglichen eine gleichmäßige Abdeckung und sind weniger anfällig für lokale Störungen, da das Signal über die gesamte Fläche gemittelt wird. Diese Eigenschaften machen flächige Sensoren ideal für Anwendungen, bei denen große Flächen oder Volumina überwacht werden müssen.

In einer alternativen Ausführungsform weisen der Messsensor und der Referenzsensor ein flächiges Sensorelement auf.

Weisen sowohl der Messsensor als auch der Referenzsensor ein flächiges Sensorelement auf, werden im Vergleich zur Nutzung bei nur einem der Sensoren, hierzu genannte Vorteile weiter verstärkt. Weiterhin bleibt die Vergleichbarkeit der Sensoren gegeben.

Eine weitere Ausführungsform sieht vor, dass der Messsensor ein allseits katalytisch beschichtetes Sensorelement aufweist.

Die allseitige Beschichtung des Sensorelemente gewährleistet eine vollständige Abdeckung, was zu einer umfassenderen und genaueren Messung führt. Durch die Beschichtung aller Seiten wird der Sensor sensitiver gegenüber dem Analyten, hier insbesondere gegenüber H2O2. Zudem reduziert die gleichmäßige Beschichtung Signalabweichungen und sorgt für konsistentere Messergebnisse, da lokale Störungen minimiert werden. Insgesamt verbessert die allseitige Beschichtung die Sensorgenauigkeit.

In einer Ausführungsform ist ein oder das Sensorelement des Messsensors in räumlicher Nähe zu einem oder dem Sensorelement des Referenzsensors angeordnet.

Wenn Sensorelemente des Mess- und Referenzsensoren in räumlicher Nähe zueinander platziert werden, sind sie identischen Umgebungsbedingungen wie Temperatur und Feuchtigkeit ausgesetzt sind. Dies minimiert Unterschiede in den Messbedingungen, erleichtert die Kalibrierung und reduziert die Auswirkungen von Störungen. Die Nähe der Sensoren führt zu konsistenteren und genaueren Messdaten, da sie unter gleichen Bedingungen arbeiten und mögliche zeitliche Verzögerungen verringert werden.

In einer Ausführungsform ist zusätzlich ein Feuchtesensor ausgebildet.

Die Kombination zusätzlicher Sensoren anderer Art, wie eines Feuchtesensors, ist vorteilhaft, da sie ergänzende Messdaten liefert und ein umfassenderes Bild der Umgebung oder des Prozesses bietet. Dies erhöht die Genauigkeit der Analyse und hilft, Fehler und Ungenauigkeiten durch Vergleich der Daten zu erkennen und zu korrigieren. Zudem verbessert die Vielfalt der Sensoren die Robustheit des Systems, indem sie die Zuverlässigkeit der Messungen sicherstellt. Insbesondere können vorliegend die Konzentration von H2O2 präziser bestimmt werden.

In einer weiteren Ausführungsform weist der Messsensor oder der Referenzsensor einen Thermistor auf.

Thermistoren können äußerst empfindlich gegenüber Temperaturänderungen sein, was eine präzise Messung und Regelung der Temperatur ermöglicht. Ihre schnelle Reaktionszeit sorgt dafür, dass sie rasch auf Temperaturveränderungen reagieren können. Darüber hinaus sind Thermistoren kompakt und passen gut in beengte Räume, was sie vorteilhaft für Platz begrenzende Anwendungen, wie in kontrollierten Umgebungen, insbesondere in Isolatoren, macht.

Ihre hohe Genauigkeit bei der Temperaturmessung ist besonders vorteilhaft, vor allem wenn es, wie vorliegend, um enge Temperaturbereiche geht. Außerdem bieten Thermistoren eine gute Langzeitstabilität und sind über lange Zeiträume zuverlässig.

In einer Ausführungsform weisen der Messsensor und der Referenzsensor einen Thermistor auf.

Weisen sowohl der Messsensor als auch der Referenzsensor einen Thermistor auf, werden im Vergleich zur Nutzung eines Thermistors bei nur einem der Sensoren, hierzu genannte Vorteile weiter verstärkt. Weiterhin bleibt die Vergleichbarkeit der Sensoren gegeben.

Bei einer vorteilhaften Ausgestaltung kann vorgesehen sein, dass zumindest der Messsensor und der Referenzsensor und optional der Feuchtesensor gemeinsam in einem Volumen mit einer größten Ausdehnung von weniger als 5cm, bevorzugt weniger als 3cm, angeordnet sind. Somit sind Messflecke von der Größe eines handelsüblichen Bioindikators erreichbar. Dies erleichtert eine unmittelbare Vergleichbarkeit mit herkömmlichen Messungen einer Biowirksamkeit als Wirksamkeitsaussage.

In einer Ausführungsform weist der Messsensor (2) und/oder der Referenzsensor (3) und/oder der Feuchtesensor (10) eine Ansprechzeit gemäß dem Standard T63, welcher die Zeit angibt bis der Messsensor (2) und/oder der Referenzsensor (3) und/oder der Feuchtesensor (10) 63% der zu messenden Messgrößendifferenz gemessen hat, von weniger als 20 Sekunden, bevorzugt von weniger als 10 Sekunden, auf.

Die kurze Ansprechzeit der Sensoren nach dem T63-Standard ermöglicht eine schnelle Erfassung von Veränderungen in der Messumgebung. Dies führt zu einer verbesserten Genauigkeit der Messungen und erlaubt eine präzisere Prozesssteuerung. Dies ist besonders vorteilhaft bei Messungen innerhalb einer geschützten Umgebung, wie beispielsweise einem Isolator, die eine zeitnahe Anpassung an wechselnde Bedingungen erfordert, wie etwa der Messung der Wasserstoffperoxid-Konzentration in Dekontaminationsprozessen. Zudem trägt die kurze Ansprechzeit der Sensoren zur Energieeffizienz bei, da Regelkreise prompter auf Änderungen reagieren können. Insgesamt führt die schnelle Ansprechzeit zu einer Optimierung von Messgenauigkeit, Prozesseffizienz und Anwendungsflexibilität.

Eine offene Abdeckung kann das erwähnte Volumen umgeben. Dies ermöglicht eine Diffusion von Messgas zu dem Messsensor und/oder dem Referenzsensor und gleichzeitig einen mechanischen Schutz.

Eine bevorzugte Anwendung sieht eine Verwendung eines erfindungsgemäßen Wasserstoffperoxidsensors, insbesondere wie zuvor beschrieben und/oder nachstehend beansprucht, in einer kontrollierten Umgebung vor. Dies ermöglicht eine einfache, lokale Überwachung und/oder Dokumentation und/oder Steuerung eines Dekontaminationsprozesses.

Bevorzugt ist die kontrollierte Umgebung ein Isolator. Somit ist eine Überwachung, Dokumentation und/oder Steuerung von Dekontaminationsprozessen in einer pharmazeutischen Fertigungsanalage möglich.

Besonders günstig ist es, wenn der Wasserstoffperoxidsensor in freistehender Anordnung betrieben wird. Somit ist eine Erfassung einer zirkulierenden oder verwirbelten Luft in der kontrollierten Umgebung möglich.

Als ein weiterer möglicherweise eigenständiger Aspekt besteht die Lösung der eingangs genannten Aufgabe in den Merkmalen des nebengeordneten Verfahrensanspruchs und somit insbesondere darin, dass bei einem Verfahren zur Bestimmung einer Konzentration von Wasserstoffperoxid in einem Messgas, wobei insbesondere das Messgas einem Messsensor und einem Referenzsensor zugeführt wird, wobei ein katalytischer Stoffumsatz des Wasserstoffperoxids an dem Messsensor thermisch erfasst wird.

Dieses Verfahren erlaubt eine präzise Bestimmung niedriger Wasserstoffperoxid Konzentrationen.

In einer Ausführungsform wird der katalytischer Stoffumsatz durch Vergleich von Temperaturmessungen am Messsensor und am Referenzsensor ermittelt.

Die Bestimmung des katalytischen Stoffumsatzes durch den Vergleich von Mess- und Referenzsignal ist vorteilhaft, weil sie die Genauigkeit der Messung erhöht. Eine präzise Referenzgröße hilft, Messfehler zu minimieren und Systemfehler zu korrigieren. Die Methode ermöglicht auch eine effektive Kalibrierung und erhöht die Zuverlässigkeit der Messung. Zudem vereinfacht der Vergleich die Interpretation der Ergebnisse und verbessert die Gesamtmessgenauigkeit.

Bei einer vorteilhaften Ausgestaltung kann vorgesehen sein, dass das Messgas eine Temperatur unterhalb 60°C aufweist. Somit ist der Verfahren bei Dekontaminationsprozessen in der pharmazeutischen Industrie einsetzbar.

Bei einer vorteilhaften Ausgestaltung kann vorgesehen sein, dass der Messsensor im Betrieb um weniger als 5K, insbesondere weniger als 1K, erwärmt wird. Somit ist ein temperatur-gleitender Messsensor realisiert und/oder ein Messsensor, dessen Eigentemperatur im Betrieb einer Umgebungstemperatur erfolgt.

Bei einer vorteilhaften Ausgestaltung der Erfindung kann vorgesehen sein, das aus einem Sensorsignal eines beheizten Sensors, insbesondere des bereits erwähnten beheizten Sensors, und bevorzugt des Referenzsensors ein Kennwert für eine Luftströmung ermittelt wird. Somit kann eine Luftströmung im Normalbetrieb einer kontrollierten Umgebung und/oder während eines Dekontaminationsprozesses einfach charakterisiert werden. Der Kennwert kann beispielsweise eine Strömungsgeschwindigkeit und/oder ein Feuchtegehalt eines Messgases sein. Beispielsweise kann aus einer Leistungsaufnahme des beheizten Sensors, seiner Eigentemperatur und einer Umgebungstemperatur eine abgeführte Wärme berechnet werden, aus der sich die Strömungsgeschwindigkeit ergibt.

In einer weiteren Ausführungsform wird ein wie oben beschriebener und/oder wie nachstehend beanspruchter Wasserstoffperoxidsensor verwendet. Dies bringt die oben genannten Vorteile, insbesondere der einzelnen Aspekte und Ausführungsformen, mit sich.

Eine vorteilhafte Verwendung des erfindungsgemäßen Wasserstoffperoxidsensors, insbesondere wie beschrieben und/oder beansprucht, ist in einer kontrollierten Umgebung, insbesondere einem Isolator. Hierbei ist es günstig, wenn der Wasserstoffperoxidsensor in freistehender Anordnung betreibbar ist, also beispielsweise außerhalb eine Förderleitung für Messgas.

Eine bevorzugte Anwendung der Erfindung sieht ein Verfahren zur Bestimmung einer Wirksamkeitsaussage einer Dekontamination mit Wasserstoffperoxid vor, bei dem eine Konzentration von Wasserstoffperoxid in einem Messgas nach einem erfindungsgemäßen Verfahren, insbesondere wie zuvor beschrieben und/oder nachstehend beansprucht, bestimmt wird und, bevorzugt in einem Erfassungsbereich des Messsensors und/oder des Referenzsensors, mit einem Feuchtesensor eine Feuchte des Messgases bestimmt wird und aus Sensorsignalen von Messsensor, Referenzsensor und Feuchtesensor eine Kenngröße für die Wirksamkeitsaussage, insbesondere ein Kill-Faktor, automatisch ermittelt wird. Somit ist eine automatische Überwachung und/oder Dokumentation und/oder Steuerung eines Dekontaminationsprozesses möglich.

Kontrollierte Umgebungen sind bekannt und können beispielsweise dadurch charakterisierbar sein, dass Zustandsparameter, beispielsweise Druck, Temperatur, Luftzusammensetzung, Luftströmungsgeschwindigkeit und/oder Luftfeuchte, und/oder ein Stoffaustausch der Umgebung mit deren Außenwelt kontrolliert definierbar ist/sind. Eine Liste von Beispielen für kontrollierte Umgebungen umfassen Containments, insbesondere Isolatoren und Gloveboxes, und Restricted Access Barrier Systems (Zugangsbeschränktes Barrieresystem, RABS), insbesondere vom offenen oder geschlossenen Typ. Kontrollierte Umgebungen werden beispielsweise eingesetzt, um während eines vorzugsweise industriellen Prozesses eine unerwünschte Wechselwirkung mit der Außenwelt zu vermindern oder zu eliminieren. Ein beispielhafter Anwendungsfall kann das Abfüllen oder Umpacken eines Medikaments sein, ein anderer eine sterile Montage eines Applikators für eine Medikament.

Die Erfindung wird nun anhand von Ausführungsbeispielen näher beschrieben, ist jedoch nicht auf die Ausführungsbeispiele beschränkt. Weitere Ausführungsbeispiele ergeben sich durch Kombination der Merkmale einzelner oder mehrerer Ansprüche untereinander und/oder mit einzelnen oder mehreren Merkmalen des Ausführungsbeispiels.

Es zeigt, in jeweils stark vereinfachter Darstellung,
- Fig. 1: eine schematische, zweidimensionale Darstellung eines Wasserstoffperoxidsensors in einer Ausführungsform,
- Fig. 2: eine schematische, zweidimensionale Darstellung einer kontrollierten Umgebung, in der ein Verfahren zur Bestimmung einer Konzentration von Wasserstoffperoxid in einem Messgas, ausgeführt wird.

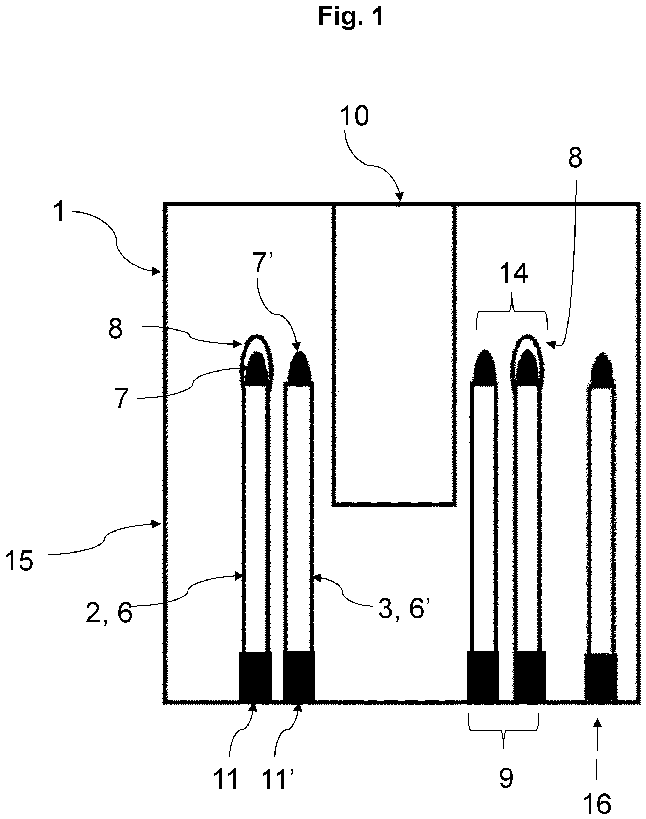

Fig. 1 zeigt einen Wasserstoffperoxidsensor 1 mit Messsensor 2 und einem Referenzsensor 3. Der Messsensor 2 und der Referenzsensor 3 ist mit einem Messgas 4 in Kontakt bringbar. Der Messsensor 2 ist dabei zu einer katalytischen Umsetzung von Wasserstoffperoxid 5 eingerichtet. Zumindest der Messsensor 2 ist als ein Temperatursensor 6 ausgebildet.

Der Wasserstoffperoxidsensor 1 ist geeignet, die exotherme Reaktion der Zersetzung von Wasserstoffperoxid in Wasser und Sauerstoff katalytisch zu unterstützen und die dabei entstehende Wärme mittels des Messsensors 2 zu detektieren. Durch die Verwendung des Referenzsensors 3 ist eine Quantifizierung und Kalibrierung der katalytischen Umsetzung, beispielsweise die Bestimmung eines absoluten Umsatzes der Reaktion, möglich. Damit kann die Konzentration von Wasserstoffperoxid in dem Messgas 4 bestimmt werden.

In Figur 1 ist der Referenzsensor 3, wie der Messsensor 2, als ein Temperatursensor 6' ausgebildet.

In einer alternativen Ausführungsform kann der Referenzsensor 3 auch geeignet sein, eine andere Messgröße des Messgases 4 zu detektieren.

Insbesondere weisen in Figur 1 der Messsensor 2 und der Referenzsensor 3 baugleiche Sensorelemente 7, 7' auf. Der Messsensor 2 ist zusätzlich mit einer katalytisch wirksamen Schicht 8 abgedeckt.

Die katalytisch wirksame Schicht 8 kann beispielsweise Mangandioxid, insbesondere mit Korngrößen zwischen 10 und 60 Mikrometern, umfassen. Das Mangandioxid kann beispielsweise auf einen Klebstoff und damit auf den Messsensor 2 aufgebracht werden. Der Klebstoff dient dabei als Träger und ist direkt auf dem Sensorelement 7 angeordnet.

In Figur 1 weisen der Messsensor 2 und der Referenzsensor 3 ein volumenhaltiges Sensorelement 7, 7' auf.

Das volumenhaltige Sensorelement 7, 7' ist in Figur 1 als elliptische Kappe an einem Ende der Sensoren 2, 3 ausgestaltet. Das volumenhaltige Sensorelement 7, 7' kann in alternativen Ausführungsformen auch andere Geometrien aufweisen.

In einer alternativen Ausführungsform kann nur einer der Sensoren 2, 3, also nur der Messsensor 2 oder nur der Referenzsensor 3, ein volumenhaltiges Sensorelement 7, 7' aufweisen.

In einer alternativen Ausführungsform können der Messsensor 2 und/oder der Referenzsensor 3 ein flächiges Sensorelement 7, 7' aufweisen. Ein solches flächiges Sensorelement 7, 7' kann auf einer oder mehreren Flächen mit einer katalytisch wirksamen Schicht 8 abgedeckt sein.

In Figur 1 weist der Messsensor 3 ein allseits katalytisch beschichtetes Sensorelement 7 auf. Mit anderen Worten sind alle Flächen oder ein Großteil der Fläche des Sensorelements 7, welche dem Messgas 4 aussetzbar oder ausgesetzt sind, mit einer, insbesondere der oben beschriebenen, katalytisch wirksamen Schicht 8 abgedeckt. Beispielsweise sind mehr als 50 %, bevorzugt mehr als 70 %, weiter bevorzugt mehr als 90 % der Fläche des Sensorelements 7, welche dem Messgas 4 aussetzbar oder ausgesetzt sind, mit einer, insbesondere der oben beschriebenen, katalytisch wirksamen Schicht 8 abgedeckt.

In Figur 1 ist ein, insbesondere das zuvor beschriebene, Sensorelement 7 des Messsensors 2 in räumlicher Nähe zu einem, insbesondere dem zuvor beschriebenen, Sensorelement 7' des Referenzsensors 3 angeordnet. In einer bevorzugten Ausführungsform sind die Sensorelemente 7 und 7' mit einem Abstand von weniger als 10 cm, weiter bevorzugt von weniger als 5 cm, insbesondere bevorzugt von weniger als 1 cm angeordnet.

Insbesondere bilden der Messsensor 2 mit dem Sensorelement 6 und der Referenzsensor 4 mit dem Sensorelement 6' in Figur 1 ein Sensorpaar 9 aus.

Zusätzlich ist der Wasserstoffperoxidsensor 1 in Figur 1 mit einem Feuchtesensor 10 ausgebildet. Der Feuchtesensor 10 kann ein kapazitiver, resistiver oder thermischer Feuchtesensor 10 sein.

In alternativen Ausführungsform kann der Wasserstoffperoxidsensor 1 zusätzlich oder alternativ einen oder mehrere Temperatursensoren und/oder einen oder mehrere H2O2-Konzentrations-sensoren umfassen. Die weiteren H2O2-Konzentrationssensoren können beispielsweise auf einem anderen physikalischen Wirkungsprinzip beruhen. In einer Ausführungsform können die weiteren H2O2-Konzentrationssensoren kapazitive Messverfahren verwenden, bei denen beispielsweise eine Polymerschicht kapazitiv überwacht wird und die Polymerschicht dem Messgas 4 (als Referenz) und einem katalytisch gefilterten Messgas (als Messgröße) ausgesetzt wird.

In Figur 1 ist der Feuchtesensor 10 zwischen zwei, insbesondere wie oben beschriebenen, Sensorpaaren 9 aus je einem Messsensor 2 und einem Referenzsensor 3 angeordnet.

Dadurch kann der Feuchtesensor 10 einer symmetrisch ausgestalteten Umgebung ausgesetzt sein. Ein Vorteil einer solchen, oder ähnlichen, Anordnung ist, dass Temperaturunterschiede oder Schwankungen gleichmäßig erfassen werden können, da sowohl der Messsensor 2 als auch der Referenzsensor 3 in ähnlicher Umgebung arbeiten. So können Einflüsse der Umgebungstemperatur auf den Feuchtesensor 10 präziser erkannt und ausgeglichen werden.

Der Messsensor 2 und/oder der Referenzsensor 3 in Figur 1 weisen einen Thermistor 11, 11' auf.

Der Thermistor 11, 11' kann als NTC- (Negative Temperature Coefficient) oder als PTC- (Positive Temperature Coefficient) Thermistor ausgestaltet sein. In einer bevorzugten Ausführungsform sind die Thermistoren 11, 11' NTC-Thermistoren, welche ihren Widerstand bei steigender Temperatur verringern.

In einer bevorzugten Ausführungsform können entsprechend schaltbare Vorwiderstände verwendet werden, deren Schaltbarkeit beispielsweise zwischen 100 Ohm und 100 kOhm liegt. Dies führt für den niedrigen Widerstand zu einem Aufheizen des Sensors, mit dem sich die Strömungsgeschwindigkeit des Messgases 4 messen lässt, wenn dessen Wärmekapazität bekannt ist.

In einer bevorzugten Ausführungsform wird ein Vorwiderstand von 100 kOhm bei einem eigenen Widerstand der Messertemperatur von 5 kOhm verwendet, so dass am Sensorelement 7, 7' nur 100-120 mV liegen, obwohl die Versorgungspannung ungefähr 3,3 V beträgt. Dies führt zu einer sehr geringen Eigen-Aufheizung des Sensorelements 7, 7'.

In einer bevorzugten Ausführungsform kann so eine Konzentration von 1 ppm Wasserstoffperoxid 5 ungefähr einen Temperaturunterschied von 1 Millikelvin aufgrund der katalytisch angeregten Stoffumsetzung bewirkt.

Der Messsensor 2 und der Referenzsensor 3 und der Feuchtesensor 10 sind gemeinsam in einem Volumen mit einer größten Ausdehnung von weniger als 5cm, bevorzugt weniger als 3cm, angeordnet.

Der Messsensor 2, der Referenzsensor 3 und der Feuchtesensor 10 weisen Ansprechzeiten gemäß dem Standard T63 auf. Die Ansprechzeit ist dabei die Geschwindigkeit, mit der der Messsensor, Referenzsensor, Feuchtesensor und/oder ein weiterer oben genannter Sensor auf Änderungen der jeweiligen Messgröße des zu messenden Mediums reagiert, beispielsweise mit der ein Temperatursensor auf Änderungen der Temperatur des zu messenden Mediums reagiert. Der Standard T63 gibt die Zeit an, bis der Sensor, beispielsweise der Temperatursensor, 63 % der zu messenden Messgrößendifferenz, beispielsweise der zu messenden Temperaturdifferenz, gemessen hat.

Eine offene Abdeckung 15 ist derart ausgebildet, beispielsweise als Gitter oder als perforierte Fläche, dass eine Diffusion von Messgas 4 zu dem Messsensor 2 und/oder dem Referenzsensor 3 stattfindet. Somit kann eine Zufuhr von Messgas ohne separate Fördereinrichtung stattfinden.

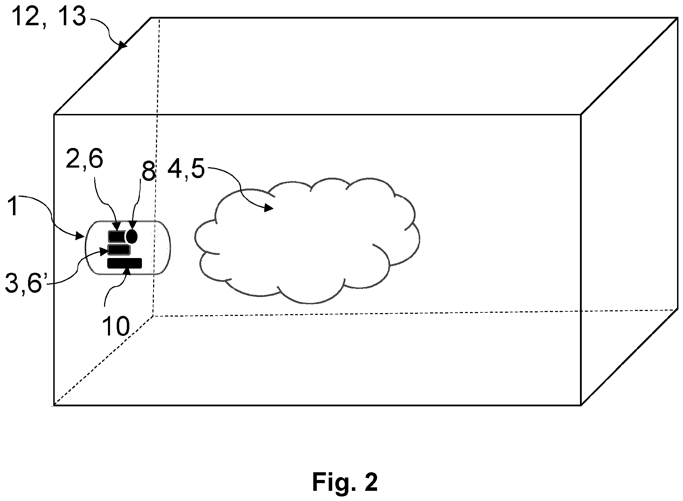

Figur 2 zeigt im Ganzen eine kontrollierte Umgebung 12. Die kontrollierte Umgebung 12 kann dabei als ein Raum oder System verstanden werden, in dem Temperatur, Luftfeuchtigkeit, Luftreinheit und andere Umweltfaktoren streng überwacht und geregelt werden, um bestimmte Bedingungen aufrechtzuerhalten. Diese Umgebungen werden genutzt, um Produkte oder Prozesse vor Verunreinigungen und äußeren Einflüssen zu schützen. Beispiele für kontrollierte Umgebungen sind Reinräume, sterile Produktionsbereiche, Klimakammern oder Isolatoren. Insbesondere kann die kontrollierte Umgebung 12 bei der vorliegenden Erfindung in einem Isolator 13 verwirklicht sein.

In der kontrollierten Umgebung 12 kann insbesondere ein Verfahren zur Bestimmung einer Konzentration von Wasserstoffperoxid 5 in einem Messgas 4 ausgeführt werden.

Das Messgas 4 diffundiert durch eine Abdeckung 15 des Wasserstoffperoxidsensors 1. Das Messgas 4 liegt bevorzugt in Umgebungstemperatur oder unterhalb von 60°C vor. Der Messsensor 2 wird so bestromt, dass er keine Erwärmung der Umgebung bewirkt.

Im Inneren des Wasserstoffperoxidsensors 1 sind die Sensorelemente 7, 7` und der Feuchtesensor 10 auf engem Bauraum beieinander, so dass insgesamt ein Volumen eingenommen wird, dass eine maximale Abmessung (hier einen Durchmesser) von weniger als 3cm hat.

Mit einem beheizbaren Sensor 16, beispielsweise einem durch einen Messstrom beheizten Sensor, kann durch Rückgriff auf eine Umgebungstemperatur des Referenzsensors 3 eine Luftströmung in an sich bekannter Weise ermittelt werden, indem eine Leistungsaufnahme mit einer erreichten Temperatur verglichen wird. Der Sensor 16 ist hierbei ebenfalls als Thermistor ausgebildet, um eine Temperaturmessung über eine Widerstandsmessung durchzuführen. Der Sensor 16 ist hierbei thermisch von den übrigen Sensoren 2, 3, 10 abgekoppelt.

Bei einem Verfahren zur Bestimmung der Konzentration von Wasserstoffperoxid 5 in einem Messgas 4 wird das Messgas 4 einem Messsensor 2 und einem Referenzsensor 3 zugeführt. Insbesondere wird ein katalytischer Stoffumsatz des Wasserstoffperoxids 5 an dem Messsensor 2 thermisch erfasst.

Insbesondere wird der katalytischer Stoffumsatz durch Vergleich von Temperaturmessungen am Messsensor 2 und am Referenzsensor 3 ermittelt.

In Figur 2 wird insbesondere ein wie oben beschriebener Wasserstoffperoxidsensor 1 verwendet, der einen Messsensor 2 mit katalytisch aktiv beschichtetem Sensorelement 7, einen Referenzsensor 3 und einen Feuchtesensor 10 umfasst.

Mit den beschriebenen Ausführungsbeispielen lässt sich ein Verfahren zur Bestimmung einer Wirksamkeitsaussage einer Dekontamination mit Wasserstoffperoxid 5, ausführen, bei dem eine Konzentration von Wasserstoffperoxid 5 in einem Messgas 4 nach einem der beschriebenen Verfahren bestimmt wird und, bevorzugt in einem Erfassungsbereich des Messsensors 2 und/oder des Referenzsensors 3, mit einem Feuchtesensor 10 eine Feuchte des Messgases 4 bestimmt wird und aus Sensorsignalen von Messsensor 2, Referenzsensor 3 und Feuchtesensor 10 eine Kenngröße für die Wirksamkeitsaussage über eine biologische Wirksamkeit der Dekontamination automatisch ermittelt wird.

Die Erfindung schlägt somit einen Wasserstoffperoxidsensor 1 vor, welcher einen Messsensor 2 und einen Referenzsensor 3 umfasst. Diese Sensoren sind mit einem Messgas in Kontakt bringbar. Der Messsensor 2 ist, beispielsweise durch eine katalytisch wirksame Schicht 8, zur katalytischen Umsetzung von Wasserstoffperoxid 5 eingerichtet. Dabei ist der Messsensor 2 als ein Temperatursensor 6 ausgebildet. Damit kann der Messsensor 2 die entstehende Wärme bei Zersetzung des Wasserstoffperoxids 5 an dem Messsensor 2 detektieren.

### Bezugszeichenliste

- 1: Wasserstoffperoxidsensor
- 2: Messsensor
- 3: Referenzsensor
- 4: Messgas
- 5: Wasserstoffperoxid
- 6: Temperatursensor als Messsensor
- 6': Temperatursensor als Referenzsensor
- 7: Sensorelement des Messsensors
- 7': Sensorelement des Referenzsensors
- 8: katalytisch wirksame Schicht
- 9: Sensorpaar
- 10: Feuchtesensor
- 11: Thermistor des Messsensors
- 11': Thermistor des Referenzsensors
- 12: kontrollierte Umgebung
- 13: Isolator
- 14: Erfassungsbereich
- 15: Abdeckung
- 16: beheizbarer Sensor

## Patentansprüche

1. Wasserstoffperoxidsensor (1), mit einem passiven Messsensor (2) und einem passiven Referenzsensor (3), wobei der Messsensor (2) und der Referenzsensor (3) mit einem Messgas (4) in Kontakt bringbar ist und wobei der Messsensor (2) zu einer katalytischen Umsetzung von Wasserstoffperoxid (5) eingerichtet ist, **dadurch gekennzeichnet, dass** zumindest der Messsensor (2) als ein Temperatursensor (6) ausgebildet ist.

2. Wasserstoffperoxidsensor (1) nach dem vorangehenden Anspruch, **dadurch gekennzeichnet, dass** der Referenzsensor (3) als ein Temperatursensor (6`) ausgebildet ist.

3. Wasserstoffperoxidsensor (1) nach einem der vorangehenden Ansprüche, **dadurch gekennzeichnet, dass** der Messsensor (2) und der Referenzsensor (3) baugleiche Sensorelemente aufweisen, insbesondere wobei der Messsensor (2) zusätzlich mit einer katalytisch wirksamen Schicht (8) abgedeckt ist

4. Wasserstoffperoxidsensor (1) nach einem der vorangehenden Ansprüche, **dadurch gekennzeichnet, dass** der Messsensor (2) und/oder der Referenzsensor (3) ein volumenhaltiges Sensorelement aufweisen.

5. Wasserstoffperoxidsensor (1) nach einem der vorangehenden Ansprüche, **dadurch gekennzeichnet, dass** der Messsensor (2) und/oder der Referenzsensor (3) ein flächiges Sensorelement aufweisen.

6. Wasserstoffperoxidsensor (1) nach einem der vorangehenden Ansprüche, **dadurch gekennzeichnet, dass** der Messsensor (2) ein allseits katalytisch beschichtetes Sensorelement aufweist.

7. Wasserstoffperoxidsensor (1) nach einem der vorangehenden Ansprüche, **dadurch gekennzeichnet, dass** ein oder das Sensorelement (7, 7`) des Messsensors (2) in räumlicher Nähe zu einem oder dem Sensorelement des Referenzsensors (3) angeordnet ist.

8. Wasserstoffperoxidsensor (1) nach einem der vorangehenden Ansprüche, **dadurch gekennzeichnet, dass** zusätzlich ein Feuchtesensor (10) ausgebildet ist.

9. Wasserstoffperoxidsensor (1) nach einem der vorangehenden Ansprüche, **dadurch gekennzeichnet, dass** der Messsensor (2) und/oder der Referenzsensor (3) einen Thermistor (11, 11`) aufweist/aufweisen.

10. Wasserstoffperoxidsensor (1) nach einem der vorangehenden Ansprüche, **dadurch gekennzeichnet, dass** zumindest der Messsensor (2) und der Referenzsensor (3) und optional der Feuchtesensor (10) gemeinsam in einem Volumen mit einer größten Ausdehnung von weniger als 5cm, bevorzugt weniger als 3cm, angeordnet sind.

11. Wasserstoffperoxidsensor (1) nach einem der vorangehenden Ansprüche, **dadurch gekennzeichnet, dass** der Messsensor (2) und/oder der Referenzsensor (3) und/oder der Feuchtesensor (10) eine Ansprechzeit gemäß dem Standard T63, welcher die Zeit angibt bis der Messsensor (2) und/oder der Referenzsensor (3) und/oder der Feuchtesensor (10) 63% der zu messenden Messgrößendifferenz gemessen hat, von weniger als 20 Sekunden, bevorzugt von weniger als 10 Sekunden, aufweist.

12. Wasserstoffperoxidsensor (1) nach einem der vorangehenden Ansprüche, **dadurch gekennzeichnet, dass** eine offene Abdeckung ausgebildet ist, insbesondere die eine Diffusion von Messgas (4) zu dem Messsensor (2) und/oder dem Referenzsensor (3) zulässt, und/oder dass ein beheizbarer Temperatursensor (16) ausgebildet ist.

13. Verwendung eines Wasserstoffperoxidsensors (1) nach einem der vorangehenden Ansprüche in einer kontrollierten Umgebung (12), insbesondere einem Isolator (12), insbesondere in freistehender Anordnung.

14. Verfahren zur Bestimmung einer Konzentration von Wasserstoffperoxid in einem Messgas (4), wobei das Messgas (4) einem passiven Messsensor (2) und einem passiven Referenzsensor (3) zugeführt wird, **dadurch gekennzeichnet, dass** ein katalytischer Stoffumsatz des Wasserstoffperoxids (5) an dem Messsensor (2) thermisch erfasst wird.

15. Verfahren nach dem vorangegangenen Anspruch, **dadurch gekennzeichnet, dass** der katalytischer Stoffumsatz durch Vergleich von Temperaturmessungen am Messsensor (2) und am Referenzsensor (3) ermittelt wird.

16. Verfahren nach einem der Ansprüche 14 bis 15, **dadurch gekennzeichnet, dass** das Messgas (4) eine Temperatur unterhalb 60°C aufweist und/oder dass der Messsensor (2) im Betrieb um weniger als 5K, insbesondere weniger als 1K, erwärmt wird.

17. Verfahren nach einem der Ansprüche 14 bis 16, **dadurch gekennzeichnet, dass** aus einem Sensorsignal eines beheizten Sensors (16) und bevorzugt des Referenzsensors (3) ein Kennwert für eine Luftströmung ermittelt wird und/oder dass ein Wasserstoffperoxidsensor (1) nach einem der Ansprüche 1 bis 11 verwendet wird.

18. Verfahren zur Bestimmung einer Wirksamkeitsaussage einer Dekontamination mit Wasserstoffperoxid (5), **dadurch gekennzeichnet, dass** eine Konzentration von Wasserstoffperoxid (5) in einem Messgas (4) nach einem Verfahren nach einem der Ansprüche 12 bis 15 bestimmt wird und, bevorzugt in einem Erfassungsbereich des Messsensors (2) und/oder des Referenzsensors (3), mit einem Feuchtesensor (10) eine Feuchte des Messgases (4) bestimmt wird und dass aus Sensorsignalen von Messsensor (2), Referenzsensor (3) und einem die Feuchtesensor (10) eine Kenngröße für die Wirksamkeitsaussage automatisch ermittelt wird.
